# EUROPEAN PATENT SPECIFICATION

(11) **EP 2 598 887 B1**
(45) Date of publication and mention of the grant of the patent: **19.08.2015**
(21) Application number: 11738220.0
(22) Date of filing: 28.07.2011
(51) Int. Cl.: G01N 33/68

(54) **METHOD FOR DETECTION OF ISCHEMIC STROKES**
VERFAHREN FÜR DEN NACHWEIS EINES ISCHÄMISCHEN SCHLAGANFALLS
PROCÉDÉ DE DÉTECTION D'ACCIDENTS ISCHÉMIQUES

(30) Priority: 28.07.2010 EP 10007854
(43) Date of publication of application: 05.06.2013
(73) Proprietor: Abbott GmbH & Co. KG, 65205 Wiesbaden-Delkenheim (DE); Fundació Hospital Universitari Vall d'Hebron - Institut de Recerca, 08035 Barcelona (ES)
(72) Inventor: CURDT, Ingo, 65239 Hochheim am Main (DE); BLINCKO, Stuart, 65207 Wiesbaden (DE); DHEIN, Jens, 65462 Ginsheim Gustavsburg (DE); DEVANARAYAN, Viswanath, Souderton, PA 18964 (US); MONTANER VILLALONGA, Joan, 08037 Barcelona (ES)
(74) Representative: Patentanwälte Gierlich & Pischitzis Partnerschaft mbB
(86) International application number: PCT/EP2011/063049
(87) International publication number: WO 2012/013758

(56) References cited:
- EP-A1- 2 019 318
- WO-A1-2008/137465
- WO-A2-2004/031242
- WO-A2-2004/059293
- WO-A2-2004/063340
- US-A1- 2005 130 230
- CHEN Y ET AL: "Neuroprotection by endogenous and exogenous PACAP following stroke", REGULATORY PEPTIDES, ELSEVIER SCIENCE BV, NL, vol. 137, no. 1-2, 15 November 2006 (2006-11-15), pages 4-19, XP025033092, ISSN: 0167-0115, DOI: 10.1016/J.REGPEP.2006.06.016 [retrieved on 2006-11-15]
- DATABASE BIOSIS [Online] BIOSCIENCES INFORMATION SERVICE, PHILADELPHIA, PA, US; March 1998 (1998-03), DULLI DOUGLAS ET AL: "Differentiation of acute cortical and subcortical ischemic stroke by risk factors and clinical examination findings", XP000002657956, Database accession no. PREV199800274552 & NEUROEPIDEMIOLOGY, vol. 17, no. 2, March 1998 (1998-03), pages 80-89, ISSN: 0251-5350
- LIJUN XU ET AL: "Overexpression of mitochondrial Hsp70/Hsp75 in rat brain protects mitochondria, reduces oxidative stress, and protects from focal ischemia", JOURNAL OF CEREBRAL BLOOD FLOW & METABOLISM, vol. 29, no. 2, 5 November 2008 (2008-11-05), pages 365-374, XP55023228, ISSN: 0271-678X, DOI: 10.1038/jcbfm.2008.125

## Description

### FIELD OF THE INVENTION

The present invention relates to the identification and use of diagnostic markers for ischemic stroke especially of lacunar subtype. The invention relates to devices and kits for performing these methods.

### BACKGROUND OF THE INVENTION

Ischemic Stroke is a manifestation of vascular injury to the brain which is commonly secondary to atherosclerosis or hypertension.

Ischemic stroke encompasses atherothrombotic, cardioembolic and lacunar types of strokes. Thrombi are occlusions of arteries created in situ within the brain, while emboli are occlusions caused by material from a distant source, such as the heart and major vessels, often dislodged due to myocardial infarct or atrial fibrillation. Less frequently, thrombi may also result from vascular inflammation due to disorders such as meningitis. Thrombi or emboli can result from atherosclerosis or other disorders, for example, arteritis, and lead to physical obstruction of arterial blood supply to the brain. Lacunar stroke refers to an infarct within non-cortical regions of the brain.

The onset of ischemic stroke is often abrupt, and can become an "evolving stroke" manifested by neurologic deficits that worsen over a 24-48 hour period. In evolving stroke, "stroke-associated symptom(s)" commonly include unilateral neurologic dysfunction that extends progressively, without producing headache or fever. Evolving stroke may also become a "completed stroke", in which symptoms develop rapidly and are maximal within a few minutes.

Ischemic stroke was thought to cause permanent injury to brain tissues. However, the similarity of the acute clinical syndromes of ischemic stroke to other diseases made it difficult to differentiate them. These are also termed "stroke mimics". Examples for such mimics are epileptic seizure, brain tumour or migraine. Therefore an ischemic stroke may remain undetected. On the other hand a falsely detected ischemic stroke may lead to a wrong treatment.

Immediate diagnosis and care of a patient experiencing stroke can be critical. For example, tissue plasminogen activator (tPA) given within three hours of symptom onset in ischemic stroke is beneficial for selected acute stroke patients. Alternatively, patients may benefit from anticoagulants (e.g., heparin) if they are not candidates for TPA therapy. Delays in the confirmation of stroke diagnosis and the identification of stroke type limit the number of patients that may benefit from early intervention therapy.

Accordingly, there is a present need in the art for a rapid, sensitive and specific diagnostic assay for stroke. Such a diagnostic assay would greatly increase the number of patients that can receive beneficial stroke treatment and therapy, and reduce the costs associated with incorrect stroke diagnosis. It is especially beneficial if the subtype of the ischemic stroke can be identified in order to adjust the treatment and/or medication.

Some diagnosis methods require expensive machines and instruments like a computer tomograph (CT). The sensitivity of these methods for ischemic stroke in the first hours after stroke is limited and therefore the additional testing of a blood bi-omarker with a better sensitivity would add diagnostic value. Stroke biochemical markers are shown in WO 2008/137465.

It is, therefore, an object of the present invention to provide a method for diagnosing ischemic stroke, preferably of lacunar subtype, especially to differentiate these ischemic strokes from stroke mimics. Further it is an object of the present invention to provide a device and a kit to carry out the methods.

This aim is achieved by the inventions as claimed in the independent claims. Advantageous embodiments are described in the dependent claims.

Even if no multiple back-referenced claims are drawn, all reasonable combinations of the features in the claims shall be disclosed.

The object of the invention is also achieved by a method. In what follows, individual steps of a method will be described in more detail. The steps do not necessarily have to be performed in the order given in the text. Also, further steps not explicitly stated may be part of the method.

The problem is solved by a method of diagnosing ischemic stroke in a subject, comprising the steps: detecting the presence and/or amount of at least one marker or at least one antibody against at least one marker in a sample from said subject, wherein the at least one marker comprises Pellino homolog 1 (Drosophila) (PELI1) and correlating the result to the occurrence or non-occurence of ischemic stroke in said subject, wherein the ischemic stroke is an ischemic stroke of lacunar subtype.

Ischemic stroke of lacunar subtype is diagnosed. The result is correlated to the occurrence or non-occurrence of a lacunar stroke (ischemic stroke of lacunar subtype) in said subject.

The methods comprise analyzing a sample obtained from a subject for the presence or amount of one or more markers or one or more antibodies against markers. These methods can comprise identifying one or more markers or antibodies, the presence or amount of which is associated with the diagnosis, prognosis, or differentiation of ischemic strokes. Once such marker(s) are identified, the level of such marker(s) in a sample obtained from a subject of interest can be measured. In certain embodiments, these markers can be compared to a level that is associated with the diagnosis, prognosis, or differentiation of ischemic strokes. By correlating the subject's marker level(s) to the diagnostic marker level(s) or the correlating with classification methods, the presence or absence of ischemic strokes and TIAs the probability of future adverse outcomes (early risk assessment, screening), etc., in a patient or apparently healthy individuum may be rapidly and accurately determined.

The method of the invention is especially suited for determining the presence or absence of a disease in a subject that is exhibiting a perceptible change in one or more physical characteristics (that is, one or more "symptoms") that are indicative of a plurality of possible etiologies underlying the observed symptom(s), one of which is ischemic stroke. These methods comprise analyzing a sample obtained from the subject for the presence or amount of one or more markers or one or more antibodies against markers selected to rule in or out stroke, or one or more types of stroke, as a possible etiology of the observed symptom(s). Etiologies other than stroke that are within the differential diagnosis of the symptom(s) observed are referred to herein as "stroke mimics". The presence or amount of such marker(s) in a sample obtained from the subject can be used to rule in or rule out stroke, preferably lacunar stroke, thereby either providing a diagnosis (rule-in) and/or excluding a diagnosis (rule-out).

The term "marker" as used herein refers to proteins or polypeptides to be used as targets for screening samples obtained from subjects. "Proteins or polypeptides" used as markers in the present invention are contemplated to include any fragments thereof, in particular, immunologically detectable fragments. One skilled in the art would recognize that minor variation of the sequence not necessarily affect the affinity of an antibody to that protein. Therefore markers that show a sequence similarity of 80 %, 85 %, 90 %, 92 %, 95 %, 97 %, 98 %, 99 % or 99.9 % to the protein sequences presented in this application are also regarded as marker as long as they bind the same antibodies. This also includes variants which differ due to posttranslational modifications such as phosphorylation or myristylation. The same is valid for nucleic acid sequences encoding the markers.

If the antibodies against the markers are to be detected the marker used for the detection of the antibodies may include modifications to the protein, which are required to perform the detection of the presence and/or the amount of the antibodies, e.g. expression as a fusion protein, e.g. as N-terminal glutathione S-transferase (GST) fusion protein. The binding of an antibody to a marker does not necessarily mean that the antibody will bind the same protein or antigen within its normal function. The method merely identifies the affinity of an antibody present in the sample to the marker in the measurement.

First, the amount of the antibody may be measured directly by the binding of the antibody against the marker, e.g. by nuclear magnetic resonance (NMR) or surface plasmon resonance.

Second, the bound antibody may be detected by the binding of another ligand specific to the bound antibody. This may be a second antibody. This ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand may also be "tagged" with one or more tags as known in the art. Such tags may then be targets for higher order ligands. Suitable tags include biotin, digoxygenin, His-Tag, Glutathion-S- Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, acridinium esters, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star(TM) (Amersham Biosciences), ECF(TM) (Amersham Biosciences). A suitable enzyme-substrate combination may result in a colored reaction product, fluorescence or chemo luminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568, Alexa 647). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme- linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex- enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The markers described herein may be used individually, or in combination with other markers.

The at least one antibody against the at least one marker as described herein can be a monoclonal or a polyclonal antibody. In a preferred embodiment the antibody is an autoantibody present in the sample. The detection of the presence and/or amount of at least one antibody in the sample corresponds to measurement of an antibody profile of the sample against the marker(s). The measurement of autoantibodies is preferred, more preferred the measurement of the amount of autoantibodies, because these autoantibodies are produced by the immune system and may be better correlated to ischemic stroke and the risk for ischemic stroke than the proteins. Furthermore the autoantibodies may be distributed better in bodily fluids since the proteins may be attached to cell surfaces.

The term "sample" as used herein refers to a sample of bodily fluid obtained for the purpose of diagnosis, prognosis, or evaluation of a subject of interest, such as a patient. In certain embodiments, such a sample may be obtained for the purpose of determining the outcome of an ongoing condition or the effect of a treatment regimen on a condition. Preferred test samples include blood, serum, plasma, cerebrospinal fluid, urine and saliva. In addition, one of skill in the art would realize that some test samples would be more readily analyzed following a fractionation or purification procedure, for example, separation of whole blood into serum or plasma components.

In a preferred embodiment of the invention the sample is a sample of blood, serum, or plasma from said subject.

The term "subject" as used herein relates to animals, preferably mammals, and, more preferably, humans.

The term "amount" as used herein encompasses the absolute amount (e.g., of a marker or an antibody against a marker), the relative amount or concentration (e.g., of a marker or an antibody against a marker) as well as any value or parameter which correlates thereto. Such values or parameters comprise intensity signal values from all specific physical or chemical properties obtained from the said markers or antibodies against a marker by direct measurements, e.g., intensity values in mass spectra or NMR spectra. Moreover, encompassed are all values or parameters which are obtained by indirect measurements specified elsewhere in this description, e.g., expression levels determined from biological read out systems in response to the peptides or intensity signals obtained from specifically bound ligands. It is to be understood that values correlating to the aforementioned amounts or parameters can also be obtained by all standard mathematical operations.

In another embodiment of the invention the presence and/or amount of at least one further marker or at least one antibody against at least one further marker in the sample is detected, wherein the at least one further marker is selected from the group consisting of Pellino homolog 1 (Drosophila) (PELI1), TNF receptor-associated protein 1 (TRAP1), Conserved oligomeric Golgi complex subunit 3 (COG3), Homo sapiens solute carrier family 38 member 3 (SLC38A3), Homo sapiens growth arrest-specific 2 like 3 (GAS2L3) and is different from the marker already measured.

These further markers may be detected together with the other markers. Groups of markers may be identified using statistical and optimizing methods for example multivariate predictive modelling, wherein statistical learning algorithms are used.

The method is also suited for acute stroke subjects. For purposes of the present invention, the term "acute stroke" refers to a stroke that has occurred within the prior 12 hours, more preferably within the prior 6 hours, and most preferably within the prior 3 hours.

Another object of the invention is the differentiation of lacunar strokes from other stroke subtypes (cardioembolic or atherothrombotic) or stroke mimics. There the same markers may be used.

In a preferred embodiment of the invention the presence and/or amount of antibodies against the at least one marker is detected, more preferably the amount. It is also possible that from the same sample presence and/or amount of a marker and antibody against the same or a different marker is determined.

In a preferred embodiment of the invention the marker is selected from the group consisting of Pellino homolog 1 (Drosophila) (PELI1), TNF receptor-associated protein 1 (TRAP1), Conserved oligomeric Golgi complex subunit 3 (COG3), Homo sapiens solute carrier family 38 member 3 (SLC38A3), Homo sapiens growth arrest-specific 2 like 3 (GAS2L3) and the presence of at least one marker or at least one antibody against at least one marker is detected and correlated to the occurrence or non-occurrence of an ischemic stroke of the lacunar subtype, preferable for the differentiation of ischemic strokes of the lacunar subtype against ischemic strokes of the cardioembolic and atherothrombotic subtype or ischemic stroke mimics.

In another preferred embodiment of the invention the marker is selected from the group consisting of Pellino homolog 1 (Drosophila) (PELI1), TNF receptor-associated protein 1 (TRAP1), Conserved oligomeric Golgi complex subunit 3 (COG3), Homo sapiens solute carrier family 38 member 3 (SLC38A3), Homo sapiens growth arrest-specific 2 like 3 (GAS2L3) and the presence of at least one marker or at least one antibody against at least one marker is detected and correlated to the occurrence or non-occurence of ischemic strokes of the lacunar subtype, preferably correlated to the occurrence or non-occurrence of ischemic strokes of the lacunar subtype against stroke mimics.

The markers can be used on their own or in combination of 2, 3, 4 or 5 markers or in combination with other markers. It is also possible to derive signatures of specific combination of markers to increase sensitivity and specificity of the method.

Another object is the use of at least one at least one marker or at least one antibody against the at least one marker in a sample from a subject, wherein the at least one marker is selected from the group consisting of Pellino homolog 1 (Drosophila) (PELI1), TNF receptor-associated protein 1 (TRAP1), Conserved oligomeric Golgi complex subunit 3 (COG3), Homo sapiens solute carrier family 38 member 3 (SLC38A3), Homo sapiens growth arrest-specific 2 like 3 (GAS2L3) for the detection of ischemic stroke, preferably for the differentiation of ischemic strokes of lacunar subtype from ischemic stroke mimics, more preferably for the differentiation of ischemic strokes of lacunar subtype from ischemic stroke mimics and ischemic stroke of the cardioembolic or atherothrombotic subtypes.

In a preferred embodiment the use also encompasses the use of the further markers for the detection of ischemic strokes. In an especially preferred embodiment the use comprises the use of at least one of the previous described markers or antibodies against these markers for the detection of ischemic strokes of lacunar subtype.

In another preferred embodiment the use encompasses the use of the markers or antibodies against the markers previously described for the method for the occurrence or non-occurrence of ischemic strokes of the ischemic stroke of the lacunar subtype for the detection of ischemic strokes of the lacunar subtype, preferably for the differentiation of ischemic strokes of the lacunar subtype against stroke mimics.

Another object is a device for the detection of ischemic strokes comprising:
a) means for determining the amount and/or presence of at least one marker or at least one antibody against at least one marker in a sample from a subject, wherein the at least one marker is selected from the group consisting of Pellino homolog 1 (Drosophila) (PELI1), TNF receptor-associated protein 1 (TRAP1), Conserved oligomeric Golgi complex subunit 3 (COG3), Homo sapiens solute carrier family 38 member 3 (SLC38A3), Homo sapiens growth arrest-specific 2 like 3 (GAS2L3); and
b) means for analyzing the amounts and/or presence of the marker(s) or antibodies against the marker(s) against a reference value and/or by classification methods.

In a preferred embodiment the device is a device for the detection of ischemic strokes of lacunar subtype, more preferably for the differentiation of ischemic strokes of lacunar subtype against stroke mimcs and ischemic strokes of cardioembolic or atherothrombotic subtypes.

In a preferred embodiment the device further comprises means for determining the amount and/or presence of the further markers or antibodies against the further markers. In an especially preferred embodiment the device comprises means for determining the amount and/or presence of at least one of the previously described markers and/or antibodies against these markers.

In a preferred embodiment the device further comprises means for determining the amount and/or presence of the previously described markers or antibodies against the markers for the method for the occurrence or non-occurrence of ischemic strokes of the ischemic stroke of the lacunar subtype.

In a preferred embodiment the means for determining the amount and/or presence are single- or multiplex detection methods, where the markers are immobilized on the solid surface. The device then comprises means to determine the amount of at least one antibody against the immobilized marker as described previously. Preferably these means are a labelled secondary antibody.

Such devices may further comprise a computer unit for analyzing and processing the measured data. Preferably a computer, which is programmed to perform classification methods or to evaluate the measurements.

In another embodiment such devices may comprise a database, wherein reference values and/or the classification methods or their parameters are stored for different analysis methods.

In another embodiment such devices may comprise displaying means to display the data.

In another embodiment the means for analyzing the measured data may analyse the data in various aspects, e.g. ischemic stroke as well as the subtype of ischemic stroke.

Preferred means for determining the amount and/or presence are disclosed in connection with embodiments relating to the method of the invention above. In such a case, the means are operatively linked in that the user of the system brings together the result of the determination of the amount and the diagnostic or prognostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or plates or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands, Plasmon surface resonance devices, NMR spectrometers, mass-spectrometers, fluorescence readers etc.) or evaluation units/devices referred to above in accordance with the method of the invention.

Another object relates to a kit adapted for carrying out the previously described method of diagnosing ischemic stroke in a subject comprising
a) means for determining the amount and/or presence of at least one marker or antibody against at least one marker in a sample from a subject, wherein the at least one marker a marker as used in the previously described method of diagnosing ischemic strokes in a subject; and
b) means for analyzing the amounts and/or presence of the at least one marker or antibodies against the at least one marker against a reference value and/or by classification methods.

In a preferred embodiment the ischemic stroke diagnosed is an ischemic stroke of lacunar subtype.

In a preferred embodiment the kit further comprises means for determining the amount and/or presence of the previously described further markers or antibodies against further markers. In a preferred embodiment the kit further comprises means for determining the amount and/or presence of the previously described markers or antibodies against the markers for the method for the occurrence or non-occurrence of ischemic strokes of the ischemic stroke of the lacunar subtype.

The means for determining the amount/or presence of antibodies against the markers may be single- or multiplex detection methods, where the markers are immobilized, e.g. on a plate or on particles on which surface the markers are placed, e.g. immobilized or present in different spots, e.g. drops of solvent. These plates may be produced by contact printing.

The term "kit" as used herein refers to a collection of the aforementioned means, preferably, provided separately or within a single container. The kit may in addition comprise means for determining the amount and/or presence of at least one marker or at least one antibody against at least one marker, preferable at least one antibody against at least one marker. Optionally, the kit may additionally comprise a user's manual for interpreting the results of any measurement(s) with respect to diagnosing ischemic stroke or its subtypes in a subject as defined in the present invention. Particularly, such manual may include information about what determined amounts and/or presences corresponds to what kind of diagnosis. This is outlined in detail elsewhere in this specification. Additionally, such user's manual may provide instructions about correctly using the components of the kit for determining the amount of the respective markers or antibodies against the respective marker.

The kit may further contain the recipes some or all of the buffers needed or may contain premixed ingredients.

The kit may further comprise a data carrier with a software for the analysis of the measured data.

Another object of the invention is the combination of any of the described methods for ruling in or out ischemic stroke with imaging methods (e.g. CT, NMR).

Another object is the use of the described method for the prediction of lacunar stroke. The markers or combination of markers described herein may also be used for the prediction of lacunar stroke.

### BRIEF DESCRIPTION OF THE FIGURES

Other objects and advantages of the present invention may be ascertained from a reading of the specification and appended claims in conjunction with the drawings and tables therein.

For a more complete understanding of the present invention, reference is established to the following description made in connection with accompanying drawings in which:
- Fig. 1:: Measured data of ischemic strokes without lacunar subtype (cardioembolic and atherothrombotic subtypes; left column), lacunar strokes and stroke mimics for marker BC018950.2 for two protoarrays.
- Fig. 2:: Measured data of ischemic strokes without lacunar subtype (cardioembolic and atherothrombotic subtypes; left column), lacunar strokes and stroke mimics for marker NM_006841.3 for two protoarrays.
- Fig. 3:: Measured data of ischemic strokes without lacunar subtype (cardioembolic and atherothrombotic subtypes; left column), lacunar strokes and stroke mimics for marker NM_020651.2 for two protoarrays.
- Fig. 4:: Measured data of ischemic strokes without lacunar subtype (cardioembolic and atherothrombotic subtypes; left column), lacunar strokes and stroke mimics for marker NM_031431.2 for two protoarrays.
- Fig. 5:: Measured data of ischemic strokes without lacunar subtype (cardioembolic and atherothrombotic subtypes; left column), lacunar strokes and stroke mimics for marker NM_174942.1 for two protoarrays.

### DETAILED DESCRIPTION OF THE INVENTION

As already described the method of diagnosing ischemic stroke in a subject, comprising the step of detecting the presence and/or amount of at least one marker or at least one antibody against at least one marker in a sample from said subject, wherein the at least one marker is selected from the group of in total 5 proteins and polypeptides. In a preferred embodiment the markers derived from proteins and polypeptides, which are encoded by the sequences with the Database number shown in the column "Sequence" in table 1. In a more preferred embodiments the markers are derived from proteins and polypeptides, which are encoded by the sequences with the database ID shown in the column "Database ID" in table 1.

In a preferred embodiment of the invention the presence and/or presence of at least one antibody against the at least one marker is detected. This is preferably done in a protein micro array, e.g. ProtoArray from Invitrogen. In such arrays the protein or polypeptide to be bound by the antibody is printed on a plate, mostly a nitrocellulose coated glass plate. It may be necessary to express the protein or polypeptide as a fusion protein, e.g. GST fusion protein. The plate is then incubated with the sample fluid, wherein the antibody (primary antibody) it so be detected. After a washing step the antibodies bound to the protein or polypeptide are detected by incubating the array with a secondary antibody, which binds the primary antibody. This secondary antibody is labelled with a detectable tag. This can be a fluorescent tag, an enzymatic tag like horseradish peroxidase, a ligand binding tag. The amount of fluorescence is proportional to the amount of primary antibody. It may be necessary to correct the value by the amount of marker present in the array.

Next to one of the markers also other markers may be measured and analyzed at the same time.

In a preferred embodiment the method is a method to distinguish ischemic stroke of lacunar subtype against other ischemic strokes and/or stroke mimics, preferably ischemic stroke of the atherothrombic and cardioembolic subtype and/or stroke mimics.

Preferred markers are shown in the tables 1 or 2. Table 2 also gives the Seq-ID-Nos of the preferred markers for the invention.

The sequences of the markers are available from public databases. The corresponding protein sequences are listed in the sequence protocol.

### Sample preparation

The measurements were preformed using the Invitrogen ProtoArray.

Samples from 12 controls (stroke mimics) and 13 ischemic stroke patients were obtained. From the stroke patients were 5 of the lacunar subtype, 4 of the cardioembolic subtype and 4 of the atherothrombotic subtype. The stroke mimics can be further classified as epileptic seizure (4 samples), Hypoglicemia (1 sample), brain tumor (3 samples), vertigo (1 sample), radial palsy (1 sample), migraine (1 sample) and syncope (1 sample).

For the measurement the ProtoArray from Invitrogen was used (www.invitrogen.com/protoarray) following the standard protocol from the manufacturer. 9500 native human proteins were expressed in baculovirus and immobilized on a chip by contact type printing. 3 additional proteins were expressed and included in the array (2 NMDA subunits). The proteins are expressed as GST fusion protein in order to allow quality control by probing the microarray with a GST-antibody.

9501 proteins (auto-antibodies against these proteins) were measured, out of which 9411 were unique. Some proteins were tested twice in different chip locations. All 9501 proteins were measured in duplicates.

For the measurement the array is incubated with the sample, optionally in a probing buffer, typically for 2 hours at 4 °C. Any antibodies present in the sample will bind to the different proteins presented in the array. The sample is decanted and the array is washed several times. Then the array is incubated with the secondary antibody. This is typically a fluorescent labelled antibody, e.g. anti-mouse or anti-rabbit Alexa Fluor 647 from Invitrogen). This incubation is also normally done for two hours and at 4 °C. The array is then washed several times and dried. Then the microarray is scanned with a fluorescent scanner (GenePix 4000B Fluorescent Scanner; Molecular Devices) and the data was acquired with GenePix Pro software (Molecular Devices) and processed using the ProtoArray Proscpector tool developed by Invitrogen, which performs the normalizing and processing of the data.

In analysing the measured data either the Minimum (Min) or Average (Avg) of the duplicates was considered.

The data was further normalized in respect to control proteins and background using either Linear Normalization (LN) or Quantile Normalization (QN).

This resulted in a total for four combinations of datasets: MinLN, MinQN, AvgLN and AvgQN.

Negative values were converted to zero. The data was further log-transformed (after adding 1 to account for zeros).

All analysis was done with all four combinations of datasets. The following results were obtained using the Minimum Quantile Normalized data (MinQN).

### Univariate Analysis

In this analysis the proteins that are significant on their own were identified using Permutation Test Statistic and Kruskal-Wallis Testing correcting for the small sample size issue. Statistical significance is reported in terms of the False Positive Rate (p-value) and the Family Wise Error Rate (FWER).

### Stroke subtypes analysis

For this analysis the data processed and normalized with the quantile normalization method was used.

In order to obtain the significant proteins the data was analyzed using the Permutation Test Statistic and Kruskal-Wallis Testing. The data of 2 samples were excluded because these samples were redraw samples from 2 patients which were taken one week after hospitalization of the patients. Only samples drawn within 3 hours after onset of the symptoms were included in analysis.

Graphs were generated for the candidates which were identified by the statistical analysis and were then visually evaluated. The 5 most promising candidate markers were selected visually.

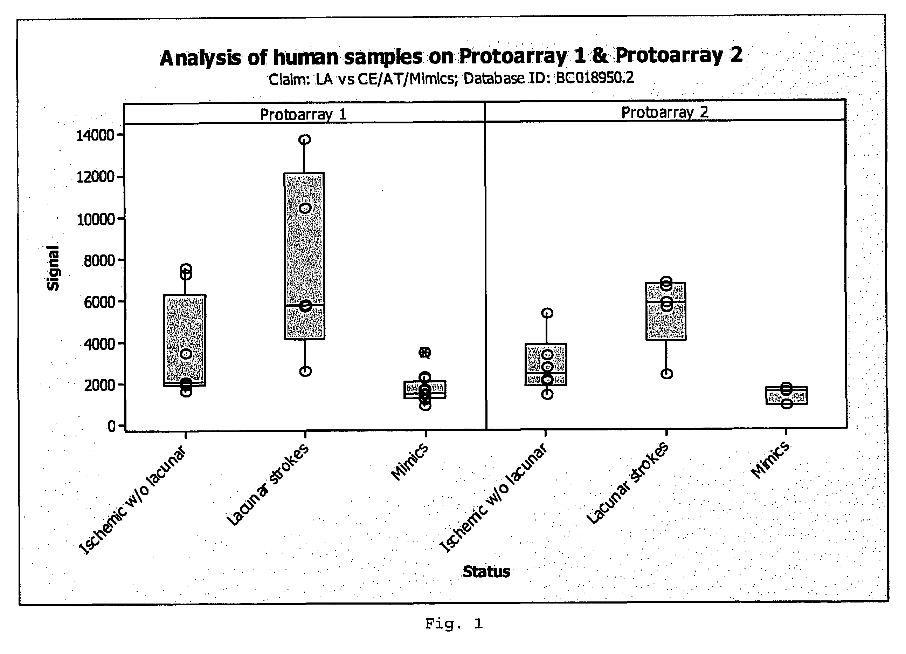

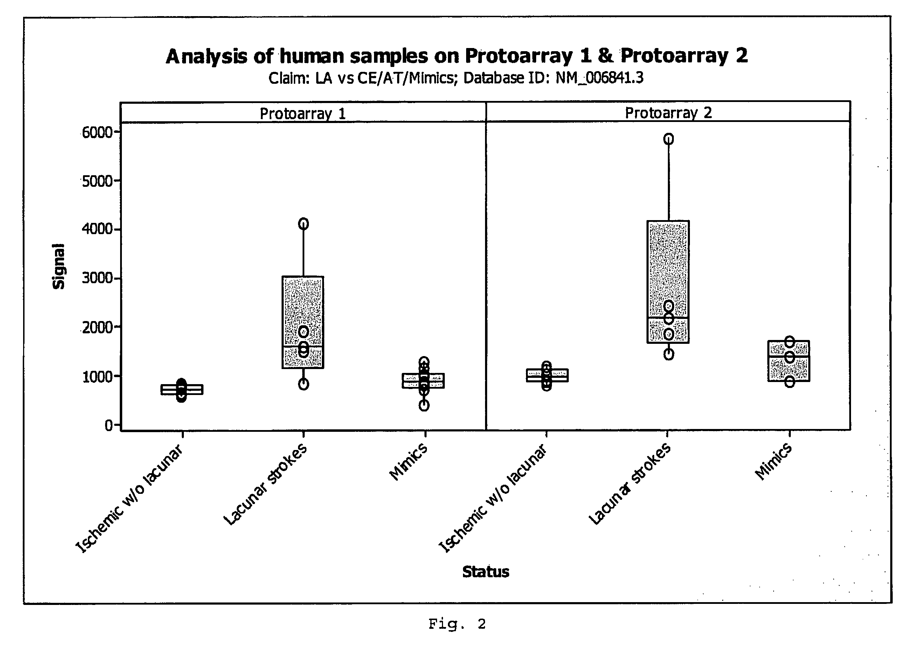

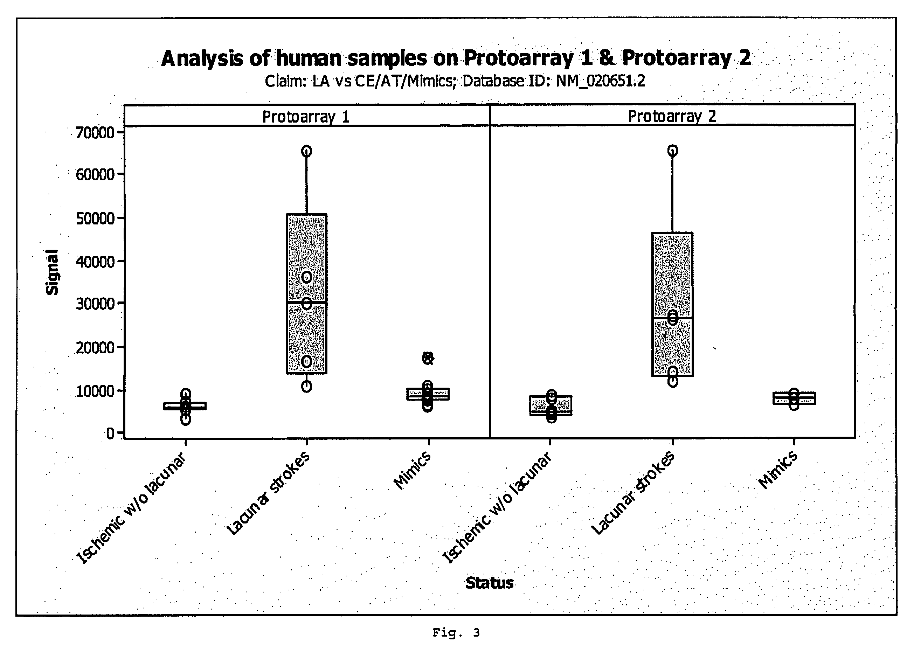

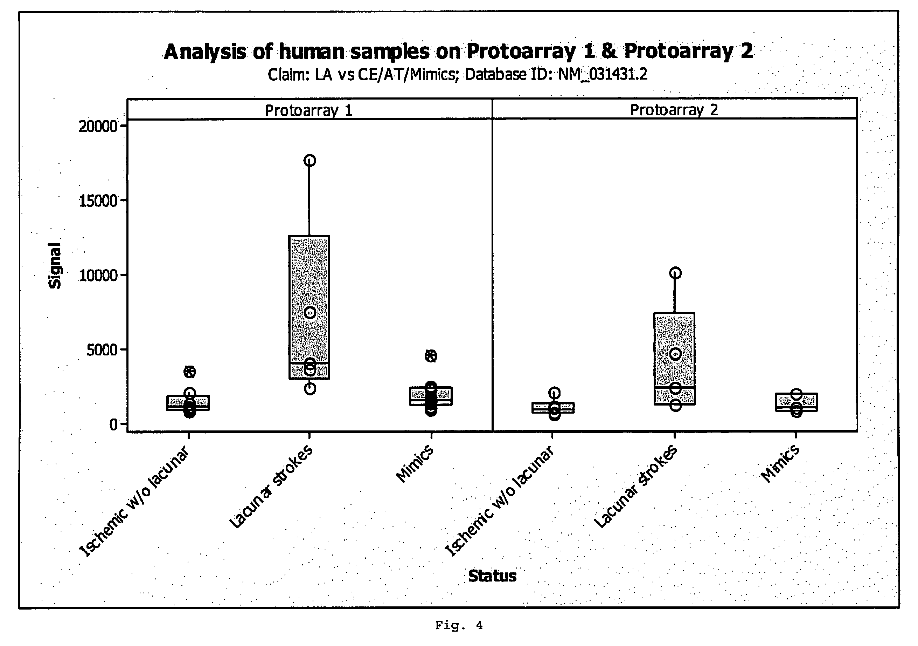

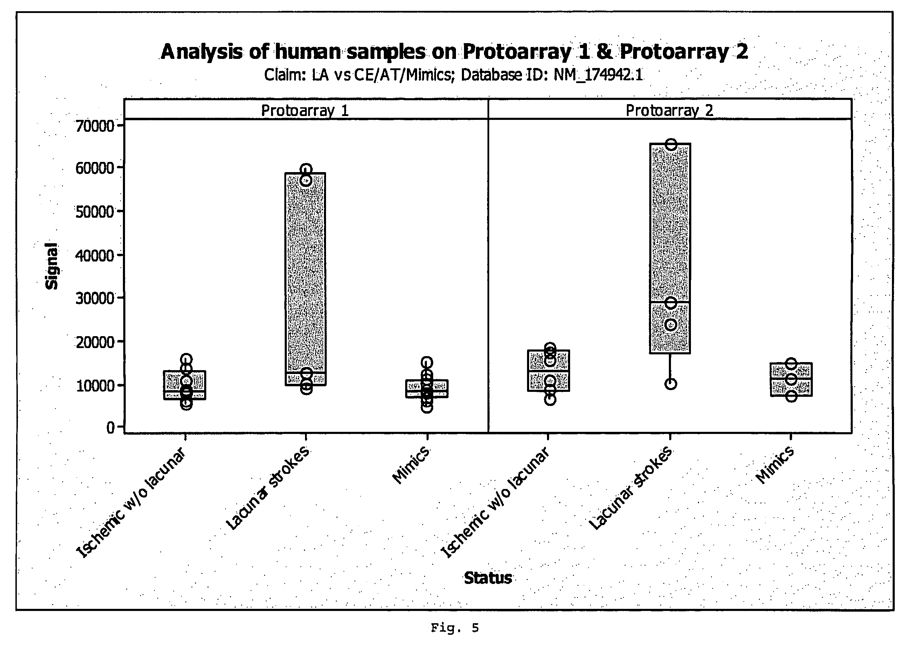

The analysis of the data in regard of stroke patients of lacunar subtype versus stroke mimics revealed 5 antibodies against proteins as marker of particular significance. These proteins are shown in table 1. The graphs of the data of these proteins are shown in figures 1 to 5.

In further analysis the data was analyze with regard to different subtypes of ischemic stroke.

The most significant proteins identified to differentiate the atherothrombic and cardioembolic subtype and stroke mimics from lacunar ischemic stroke are shown in table 1. The corresponding graphs are displayed in figures 1 to 5.

While the present inventions have been described and illustrated in conjunction with a number of specific embodiments, those skilled in the art will appreciate that variations and modifications may be made without departing from the principles of the inventions as herein illustrated, as described and claimed. The present inventions may be embodied in other specific forms without departing from their spirit or essential characteristics. The described embodiments are considered in all respects to be illustrative and not restrictive. The scope of the inventions are, therefore, indicated by the appended claims, rather than by the foregoing description. All changes which come within the meaning and range of equivalence of the claims are to be embraced within their scope.

**Table 1**

| **#** | **Sequence** | **Database ID** | **Description** |
|---|---|---|---|
| 1 | BC018950 | BC018950.2 | TNF receptor-associated protein 1 (TRAP1) |
| 2 | NM_020651 | NM_020651.2 | Pellino homolog 1 (Drosophila) (PELI1) (IOH26519) |
| 3 | NM_031431 | NM_031431.2 | Conserved oligomeric Golgi complex subunit 3 (COG3) |
| 4 | NM_006641 | NM_006841.3 | Homo sapiens solute carrier family 38, member 3 (SLC38A3) |
| 5 | NM_174942 | NM_174942.1 | Homo sapiens growth arrest-specific 2 like 3 (GAS2L3) |

**Table 2**

| Accession Number | Seq-ID-No. | Length (amino acids) |
|---|---|---|
| BC018950.2 | 1 | 704 |
| NM 020651.2 | 2 | 418 |
| NM 031431.2 | 3 | 828 |
| NM 006841.3 | 4 | 504 |
| NM 174942.1 | 5 | 694 |

## Claims

1. A method of diagnosing ischemic stroke in a subject, comprising the steps: detecting the presence and/or amount of at least one marker or at least one antibody against at least one marker in a sample from said subject, wherein the at least one marker comprises Pellino homolog 1 (Drosophila) (PELI1);
and correlating the result to the occurrence or non-occurence of ischemic stroke in said subject, wherein the ischemic stroke is an ischemic stroke of lacunar subtype.

2. The method of claim 1, wherein the sample is a sample of blood, serum and/or plasma from said subject.

3. The method of one of the claims 1 or 2, wherein the presence and/or amount of at least one antibody against the at least one marker is detected.

4. Any of the previously claimed methods for ruling in or out ischemic stroke in combination with imaging methods (e.g. CT, NMR).

## Patentansprüche

1. Verfahren zur Diagnose von ischämischem Schlaganfall bei einem Patienten, umfassend die Schritte: Nachweisen des Vorliegens und/oder der Menge wenigstens eines Markers oder wenigstens eines Antikörpers gegen wenigstens einen Marker in einer Probe von dem Patienten, wobei der wenigstens eine Marker Pellino-Homolog 1 (Drosophila) (PELI1) umfasst;
und Korrelieren des Ergebnisses mit dem Auftreten oder Nichtauftreten von ischämischem Schlaganfall bei dem Patienten, wobei es sich bei dem ischämischen Schlaganfall um einen ischämischen Schlaganfall des lakunären Subtyps handelt.

2. Verfahren nach Anspruch 1, wobei es sich bei der Probe um eine Probe von Blut, Serum und/oder Plasma aus dem Patienten handelt.

3. Verfahren nach einem der Ansprüche 1 oder 2, wobei das Vorliegen und/oder die Menge wenigstens eines Antikörpers gegen den wenigstens einen Marker nachgewiesen wird.

4. Eines der zuvor beanspruchten Verfahren zum Bestätigen oder Ausschließen von ischämischem Schlaganfall in Kombination mit bildgebenden Verfahren (z.B. CT, NMR).

## Revendications

1. Procédé de diagnostic d'accident ischémique cérébral chez un sujet, comprenant les étapes de :
détection de la présence et/ou la quantité d'au moins un marqueur ou au moins d'un anticorps contre au moins un marqueur dans un échantillon dudit sujet, dans lequel l'au moins un marqueur comprend l'homologue 1 de Pellino (Drosophile) (PELI1) ;
et corrélation du résultat à l'occurrence ou non occurrence d'un accident ischémique cérébral chez ledit sujet, dans lequel l'accident ischémique cérébral est un accident ischémique cérébral de sous-type lacunaire.

2. Procédé de la revendication 1, dans lequel l'échantillon est un échantillon de sang, de sérum et/ou de plasma dudit sujet.

3. Procédé d'une des revendications 1 ou 2, dans lequel la présence et/ou la quantité d'au moins un anticorps contre l'au moins un marqueur sont détectées.

4. Procédé de l'une quelconque des revendications précédentes pour confirmer ou exclure un accident ischémique cérébral en combinaison avec des méthodes d'imagerie (par exemple, TDM, RMN).
